# EUROPEAN PATENT APPLICATION

(11) **EP 4 194 854 A1**
(43) Date of publication of application: **14.06.2023**
(21) Application number: 21890371.4
(22) Date of filing: 17.12.2021
(51) Int. Cl.: G01N 33/52, A61B 10/00

(54) **SAMPLE COLLECTION AND TEST CARD**

(30) Priority: 14.10.2021 CN 202111199721
(71) Applicant: Shenzhen Watmind Medical Co., Ltd., Shenzhen Guangdong 518000 (CN)
(72) Inventor: WANG, Dong, Shenzhen, Guangdong 518000 (CN); JIANG, Rongxiang, Shenzhen, Guangdong 518000 (CN); HUANG, Kailing, Shenzhen, Guangdong 518000 (CN); MAO, Qianqian, Shenzhen, Guangdong 518000 (CN); LU, Fangbao, Shenzhen, Guangdong 518000 (CN)
(74) Representative: Petraz, Gilberto Luigi
(86) International application number: PCT/CN2021/139034
(87) International publication number: WO 2023/060758

(57) **Abstract**

The present invention is applicable to the medical field, and provides a sample collection and detection card. A lower cover of the card is provided with a sample loading chamber capable of accommodating a sampling part of a sampling end, an outlet is arranged at a bottom of the sample loading chamber, and a sample loading area of a test strip is located at the outlet; a fixing structure for fixing a sampling rod to a box is further arranged between the box and the sampling rod, and the sampling part is deformable when the sampling rod is fixed to the box. In embodiments of the present invention, the sample loading chamber is arranged on the lower cover, and the sample loading area of the test strip is arranged at the outlet of the sample loading chamber, so that the sample loading chamber is in direct contact with the sample loading area of the test strip, samples are in direct contact with the sample loading area, and thus a required sample size can be reduced. Therefore, a volume of the sampling part of the sampling rod can be reduced, and an overall volume of the sampling end of the sampling rod is smaller. Sampling time of a user is significantly reduced, the user feels more comfortable, and user experience is improved.

## Description

### TECHNICAL FIELD

The present invention relates to the medical field, and in particular, to a sample collection and detection card.

### BACKGROUND

Various tests are usually required for diagnosis of diseases, and samples to be tested need to be collected before the tests are carried out. The utility model No. CN210401428U discloses a saliva collection and detection apparatus, including an upper cover, a lower cover, a sampling rod and a test strip. A saliva absorption pad is located below a rod head of the sampling rod. A sample injection window is disposed on the upper cover, a pressing plate is disposed on one side of the sample injection window, a plurality of transverse ribs are arranged on the pressing plate, and a saliva inlet hole is formed beside the pressing plate. A saliva storage space is further formed in the upper cover and located beside the saliva inlet hole, and a residual saliva collection area is formed in a corresponding area of the lower cover.

The saliva storage space is formed above the upper cover, the upper cover has a certain thickness, and a space is formed between the upper cover and the lower cover. The test strip is not in direct contact with the saliva storage space, and the residual saliva collection area is formed in the corresponding area of the lower cover. Some saliva samples flow to the test strip to be absorbed by the test strip, and the other samples flow into the residual saliva collection area. Therefore, the sampling rod needs to collect more saliva samples, so that the saliva absorption pad at the sampling end of the sampling rod has a large volume. When the saliva samples are collected, the saliva absorption pad is put into the mouth of a user for a long time, which makes the user feel uncomfortable and have poor user experience.

### SUMMARY

Embodiments of the present invention provide a sample collection and detection card, aiming to resolve a problem of large volume of a sampling end of a sampling rod.

The embodiments of the present invention are implemented as follows: a sample collection and detection card, including a box and a sampling rod, where the box includes an upper cover and a lower cover, a test strip is arranged between the upper cover and the lower cover, the upper cover is provided with an observation window and an injection port, one end of the sampling rod is a sampling end, and the other end is a handle end,
the lower cover is provided with a sample loading chamber capable of accommodating a sampling part of the sampling end, an outlet is arranged at a bottom of the sample loading chamber, and a sample loading area of the test strip is located at the outlet; and
a fixing structure for fixing the sampling rod to the box is further arranged between the box and the sampling rod, and the sampling part is deformable when the sampling rod is fixed to the box.

As a development, a periphery of the bottom of the sample loading chamber is higher than the outlet.

As a development, the sample loading area of the test strip is located in the outlet.

As a development, a top surface of the sample loading area of the test strip is flush with a bottom surface of the sample loading chamber.

As a development, the sampling end of the sampling rod is provided with a display part, and the sampling part is arranged at a lower side of the display part.

As a development, the display part includes an indicator, and the indicator is capable of changing color in the presence of water.

As a development, the sampling part includes an absorbent arranged at a bottom of the sampling end.

As a development, the fixing structure includes:
an insertion hole and a first elastic inverted buckle arranged on two opposite side walls of the sample loading chamber respectively; and
a first limit insert block capable of being inserted into the insertion hole and a limit buckle in a hook-and-buckle fit with the first elastic inverted buckle, which are arranged on two opposite side walls of the sampling end.

As a development, the fixing structure includes:
a second limit insert block capable of being inserted into an inner side of the upper cover and a second elastic inverted buckle in a hook-and-buckle fit with the inner side of the upper cover, which are arranged on two opposite side walls of the sampling end.

As a development, a top of the sample loading chamber is flush with or lower than a top surface of the upper cover.

In embodiments of the present invention, the sample loading chamber is arranged on the lower cover, and the sample loading area of the test strip is arranged at the outlet of the sample loading chamber, so that the sample loading chamber is in direct contact with the sample loading area of the test strip, samples are in direct contact with the sample loading area, and thus a required sample size can be reduced. Therefore, a volume of the sampling part of the sampling rod can be reduced, and an overall volume of the sampling end of the sampling rod is smaller. Sampling time of a user is significantly reduced, the user feels more comfortable, and user experience is improved.

### BRIEF DESCRIPTION OF DRAWINGS

FIG. 1 is a perspective view according to an embodiment of the present invention;
FIG. 2 is an exploded view according to an embodiment of the present invention;
FIG. 3 is a sectional view according to an embodiment of the present invention;
FIG. 4 is an assembly view of an upper cover and a lower cover according to an embodiment of the present invention;
FIG. 5 is an assembly view of a sampling rod and a lower cover according to an embodiment of the present invention;
FIG. 6 is an assembly view of a sampling rod and a lower cover from another perspective according to an embodiment of the present invention;
FIG. 7 is a perspective view of a lower cover according to an embodiment of the present invention; and
FIG. 8 is a perspective view of a sampling rod according to an embodiment of the present invention.

In the figures, 10. Sampling rod; 11. Sampling end; 111. Sampling part; 112. First limit insert block; 113. Limit buckle; 114. Display part; 12. Handle end; 20. Upper cover; 21. Observation window; 22. Injection port; 30. Lower cover; 31. Sample loading chamber; 311. Outlet; 312. Insertion hole; 313. First elastic inverted buckle; 32. Test paper slot; 40. Test strip; and 41. Sample loading area.

### DESCRIPTION OF EMBODIMENTS

To make the objectives, technical solutions, and advantages of the present invention clearer, the present invention will be further described in detail below with reference to the accompanying drawings and embodiments. It should be understood that specific embodiments described herein are merely intended to explain the present invention, and are not intended to limit the present invention.

In the sample collection and detection card according to the embodiments of the present invention, a lower cover of the card is provided with a sample loading chamber capable of accommodating a sampling part of the sampling end, an outlet is arranged at a bottom of the sample loading chamber, and a sample loading area of the test strip is located at the outlet; a fixing structure for fixing the sampling rod to the box is further arranged between the box and the sampling rod, and the sampling part is deformable when the sampling rod is fixed to the box. In embodiments of the present invention, the sample loading chamber is arranged on the lower cover, and the sample loading area of the test strip is arranged at the outlet of the sample loading chamber, so that the sample loading chamber is in direct contact with the sample loading area of the test strip, samples are in direct contact with the sample loading area, and thus a required sample size can be reduced. Therefore, a volume of the sampling part of the sampling rod can be reduced, and an overall volume of the sampling end of the sampling rod is smaller. Sampling time of a user is significantly reduced, the user feels more comfortable, and user experience is improved.

### Embodiment 1

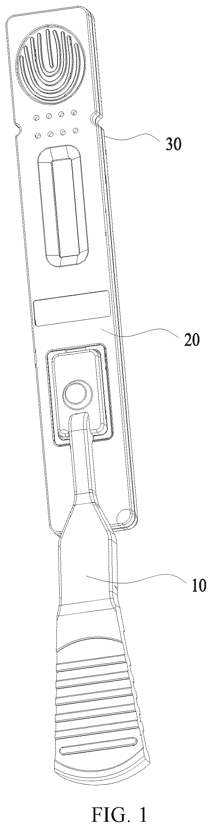

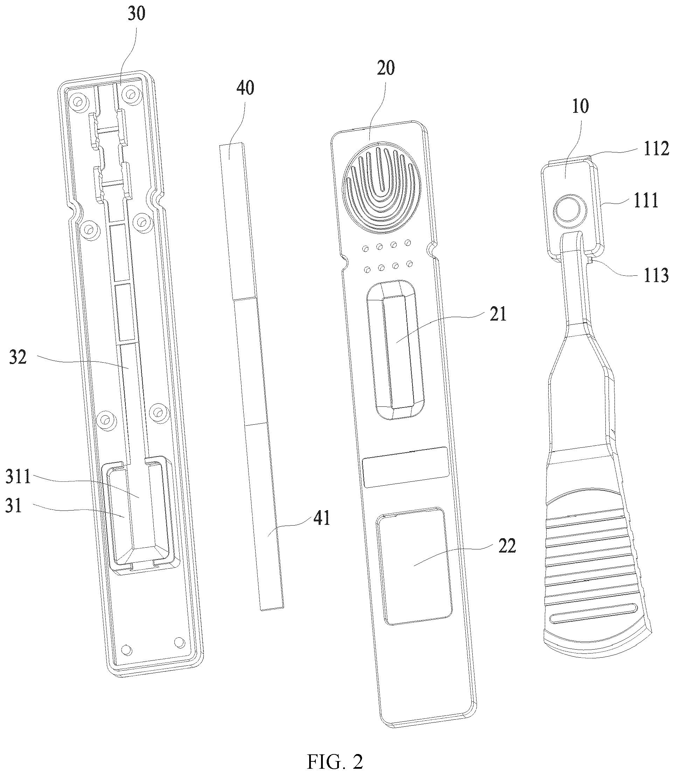

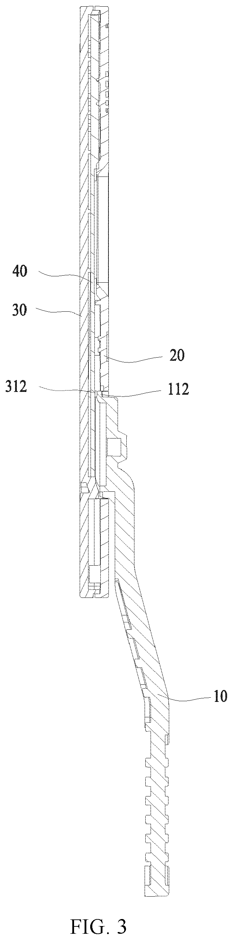

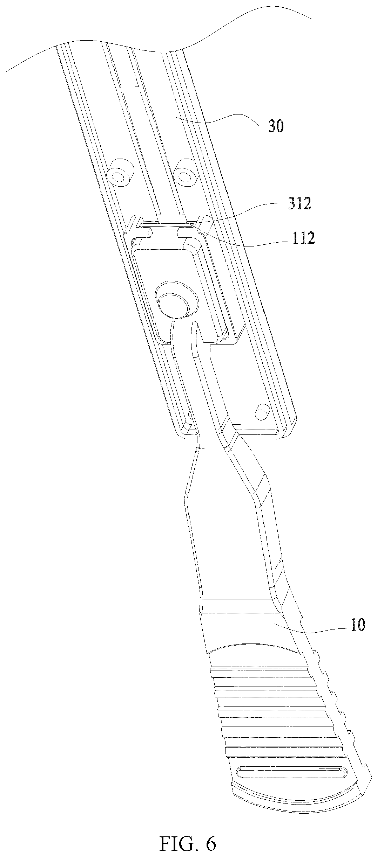

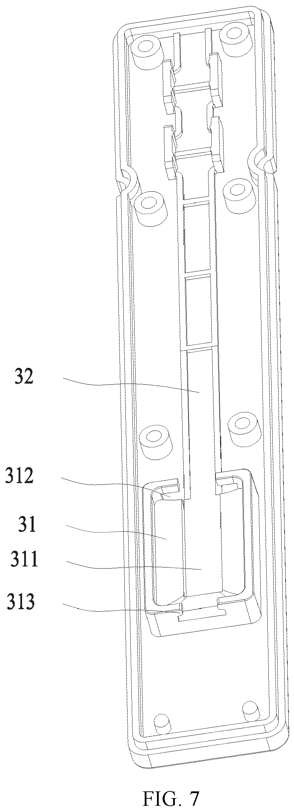

As shown in FIG. 1 to FIG. 8, the present invention provides a sample collection and detection card, including a box and a sampling rod 10, where the box includes an upper cover 20 and a lower cover 30, a test strip 40 is arranged between the upper cover 20 and the lower cover 30, the upper cover 20 is provided with an observation window 21 and an injection port 22, one end of the sampling rod 10 is a sampling end 11, and the other end is a handle end 12;
the lower cover 30 is provided with a sample loading chamber 31 capable of accommodating a sampling part 111 of the sampling end 11, an outlet 311 is arranged at a bottom of the sample loading chamber 31, and a sample loading area 41 of the test strip 40 is located at the outlet 311; and
a fixing structure for fixing the sampling rod 10 to the box is further arranged between the box and the sampling rod 10, and the sampling part 111 is deformable when the sampling rod 10 is fixed to the box.

The sampling part 111 of the sampling end 11 is arranged at a bottom of the sampling end 11, and the sampling end 11 may be the sampling part 111. The injection port 22 is located above the sample loading chamber 31, the sampling part 111 of the sampling end 11 may enter the sample loading chamber 31 through the injection port 22, or the sample loading chamber 31 extends into the injection port 22, and the sampling part 111 of the sampling end 11 may directly enter the sample loading chamber 31. A test paper slot 32 for placing the test paper 40 is formed on the lower cover 30.

The outlet 311 at the bottom of the sample loading chamber 31 may be provided at any position at the bottom of the sample loading chamber 31, and optionally, the outlet 311 may be provided at a center of the bottom of the sample loading chamber 31.

In another optional embodiment of the present invention, a periphery of the bottom of the sample loading chamber 31 is higher than the outlet 311. Specifically, a bottom surface of the sample loading chamber 31 may be arranged as an arc surface with an arc bottom at the outlet 311, and the bottom surface of the sample loading chamber 31 may be arranged as an oblique plane with a bottom at the outlet 311. When the sampling part 111 is deformed to extrude a sample, the sample flows downward to the outlet 311 arranged at the bottom of the sample loading chamber 31, so as to allow the sample to flow to the sample loading area 41.

The sample loading area 41 of the test strip 40 is located at the outlet 311, that is, the sample loading area 41 of the test strip 40 may be located in the outlet 311 or at a bottom of the outlet 311, or enter the sample loading chamber 31 through the outlet 311. When the sample loading area 41 of the test strip 40 is located in the outlet 311, the test strip 40 can be closer to the sampling part 111 at the bottom of the sampling end 11. Further, when a top surface of the sample loading area 41 of the test strip 40 is flush with the bottom surface of the sample loading chamber 31, or when the sample loading area 41 enters the sample loading chamber 31 through the outlet 311, the sampling part 111 can be directly squeezed against the sample loading area 41 of the test strip 40 to allow the sample to directly enter the sample loading area 41, so that a sample size of samples that need to be absorbed by the sampling part 111 is smaller.

The sampling part 111 includes an absorbent arranged at the bottom of the sampling end 11. The absorbent is used to absorb a liquid sample during sample collection. The absorbent is made of a soft material such as a sponge and gauze, is arranged at the bottom of the sampling end 11, and is deformable under the action of an external force. The sampling part 111 may be entirely made up of an absorbent, or the sampling part 111 may be made up of a support mounted at the bottom of the sampling end 11 and an absorbent mounted on the support.

The sampling part 111 is deformable when the sampling rod 10 is fixed to the box. Specifically, when the sampling rod 10 is fixed to the box, the sampling part 111 is deformed only by making the sampling part 111 interfere with a side wall and/or bottom wall of the sample loading chamber 31, and squeezing the sampling part 111 against the side wall and/or the bottom wall of the sample loading chamber 31. The sampling part 111 may interfere with the side wall and/or the bottom wall of the sample loading chamber 31 only by designing sizes of the sampling part 111 and the sample loading chamber 31 or designing a location and a size of the fixing structure.

In the present invention, after the sampling part 111 of the sampling rod 10 absorbs samples, the sampling rod 10 is fixed to the box, so that the sampling part 111 is squeezed against the side wall and/or the bottom wall of the sample loading chamber 31 to deform the sampling part 111, and the samples absorbed by the sampling part 111 are gradually squeezed into the sample loading chamber 31. Because the sample loading area 41 of the test strip 40 is arranged at the outlet 311 at the bottom of the sample loading chamber 31, the sample loading chamber 31 is closer to the sample loading area 41 of the test strip 40 to allow the samples to enter the sample loading area 41 directly, so that all the samples in the sample loading chamber 31 can flow to the sample loading area 41 without reserving extra samples, and the sample size of sample that need to be absorbed by the sampling part 111 is also small. Therefore, a volume of the sampling part 111 can be reduced, and an overall volume of the sampling end 11 of the sampling rod 10 is smaller.

In the embodiments of the present invention, the sample loading chamber 31 is arranged on the lower cover 30, and the sample loading area 41 of the test strip 40 is arranged at the outlet 311 of the sample loading chamber 31, so that the sample loading chamber 31 is in direct contact with the sample loading area 41 of the test strip 40, and samples are in direct contact with the sample loading area 41, and thus a required sample size can be reduced. Therefore, a volume of the sampling part 111 of the sampling rod 10 can be reduced, and an overall volume of the sampling end 11 of the sampling rod 10 is smaller. Sampling time of a user is significantly reduced, the user feels more comfortable, and user experience is improved.

### Embodiment 2

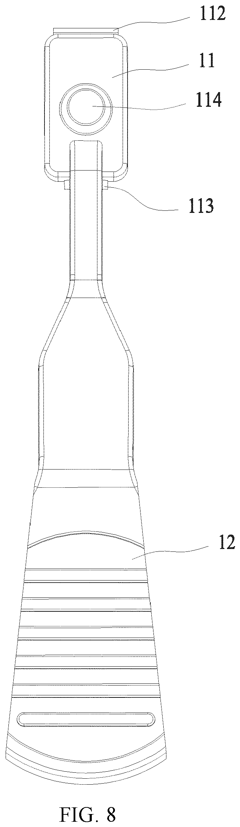

As shown in FIG. 8, in an optional embodiment of the present invention, the sampling end 11 of the sampling rod 10 is provided with a display part 114, and the sampling part 111 is arranged at a lower side of the display part 114. The display part 114 is configured to indicate whether the sampling part 111 has collected enough samples. When the sampling part 111 has collected enough samples, the display part will give a prompt instruction, such as a color change, a sound or light.

Specifically, the display part includes an indicator, and the indicator is capable of changing color in the presence of water. The indicator includes, but is not limited to, cupric sulfate anhydrous, allochroic silica gel and color-changing pigments (such as lemon yellow), and the indicator is capable of changing color in the presence of water. The indicator may be loaded directly into the sampling rod 10. The display part may further include a kit in which the indicator is placed, and the kit is mounted in the sampling rod 10.

### Embodiment 3

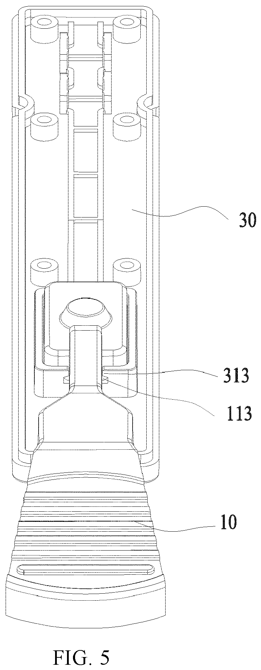

As shown in FIG. 5 to FIG. 7, in an optional embodiment of the present invention, the fixing structure includes:
an insertion hole 312 and a first elastic inverted buckle 313 arranged on two opposite side walls of the sample loading chamber 31 respectively; and
a first limit insert block 112 capable of being inserted into the insertion hole 312 and a limit buckle 113 in a hook-and-buckle fit with the first elastic inverted buckle 313, which are arranged on two opposite side walls of the sampling end 11.

In the embodiment of the present invention, the insertion hole 312 may be a through hole or a blind hole in a side wall of the sample loading chamber 31, and the first limit insert block 112 may be a protruding block on a side wall of the sampling end 11. The first limit insert block 112 has overall dimensions slightly smaller than those of the insertion hole 312, so that the first limit insert block 112 may be inserted into the insertion hole 312, and a location of the first limit insert block 112 is limited by a top wall of the insertion hole 312. Locations of the first elastic inverted buckle 313 and the limit buckle 113 may be further interchangeable, which is an equivalent solution of the present invention and falls within the protection scope of the present invention. To fix the sampling rod 10 to the box, the first limit insert block 112 is inserted into the insertion hole 312 first, then the limit buckle 113 is placed above a side wall of the sample loading chamber 31, and finally the handle end 12 of the sampling rod 10 is pressed hard to elastically deform the first elastic inverted buckle 313, and snap the limit buckle 113 into the first elastic inverted buckle 313 for a hook-and-buckle fit, so that the sampling rod 10 is fixed to the box.

In another optional embodiment of the present invention, the test paper slot 32 runs through a side wall of the sample loading chamber 31, and the test strip 40 can be placed directly into the test paper slot 32 from above the sample loading chamber 31 to facilitate installation of the test strip 40.

### Embodiment 4

In an optional embodiment of the present invention, the fixing structure includes:
a second limit insert block capable of being inserted into an inner side of the upper cover 20 and a second elastic inverted buckle in a hook-and-buckle fit with the inner side of the upper cover 20, which are arranged on two opposite side walls of the sampling end 11.

In this embodiment, the inner side of the upper cover 20 plays a role of the insertion hole 312 and the limit buckle 113 in Embodiment 3, and the inner side of the upper cover 20 can limit a location of the second limit insert block and is in a hook-and-buckle fit with the second elastic inverted buckle. A specific usage method is the same as that in Embodiment 3, and details are not described herein again.

### Embodiment 5

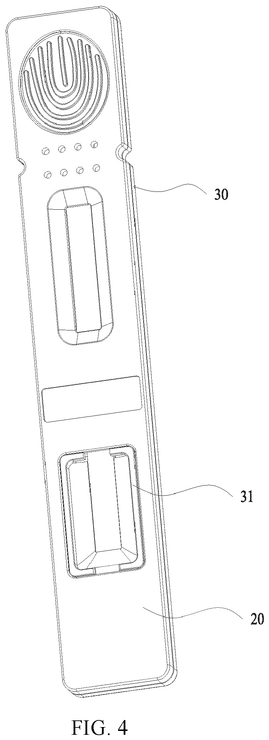

As shown in FIG. 4, in an optional embodiment of the present invention, a top of the sample loading chamber 31 is flush with or lower than a top surface of the upper cover 20.

In this embodiment, the top of the sample loading chamber 31 is flush with or lower than the top surface of the upper cover 20, then the top of the sample loading chamber 31 does not protrude from the top surface of the upper cover 20, and the top surface of the upper cover 20 is flat. The simplest way to install the upper cover 20 and the lower cover 30 is to squeeze and deform a snap hook and then snap the snap hook into a buckle through a hook-and-buckle fit. In the present invention, the top surface of the upper cover 20 is flat, and the upper cover 20 may be in press fit with the lower cover 30 by rolling with a roller, and the snap hook is squeezed and deformed and then snapped into the buckle for a hook-and-buckle fit. An automatic production device can be used for rolling with the roller without manual press fit, so that production efficiency can be improved, and labor costs are reduced.

The foregoing descriptions are merely preferred embodiments of the present invention and are not intended to limit the present invention. Any modification, equivalent replacement, improvement and the like made within the spirit and principle of the present invention shall fall within the protection scope of the present invention.

## Claims

1. A sample collection and detection card, comprising a box and a sampling rod, wherein the box comprises an upper cover and a lower cover, a test strip is arranged between the upper cover and the lower cover, the upper cover is provided with an observation window and an injection port, one end of the sampling rod is a sampling end, and the other end is a handle end,
wherein the lower cover is provided with a sample loading chamber capable of accommodating a sampling part of the sampling end, an outlet is arranged at a bottom of the sample loading chamber, and a sample loading area of the test strip is located at the outlet; and
wherein a fixing structure for fixing the sampling rod to the box is further arranged between the box and the sampling rod, and the sampling part is deformable when the sampling rod is fixed to the box.

2. The sample collection and detection card according to claim 1, wherein a periphery of the bottom of the sample loading chamber is higher than the outlet.

3. The sample collection and detection card according to claim 1, wherein the sample loading area of the test strip is located in the outlet.

4. The sample collection and detection card according to claim 3, wherein a top surface of the sample loading area of the test strip is flush with a bottom surface of the sample loading chamber.

5. The sample collection and detection card according to claim 1, wherein the sampling end of the sampling rod is provided with a display part, and the sampling part is arranged at a lower side of the display part.

6. The sample collection and detection card according to claim 5, wherein the display part comprises an indicator, and the indicator is capable of changing color in the presence of water.

7. The sample collection and detection card according to claim 5, wherein the sampling part comprises an absorbent arranged at a bottom of the sampling end.

8. The sample collection and detection card according to claim 1, wherein the fixing structure comprises:
an insertion hole and a first elastic inverted buckle arranged on two opposite side walls of the sample loading chamber respectively; and
a first limit insert block capable of being inserted into the insertion hole and a limit buckle in a hook-and-buckle fit with the first elastic inverted buckle, which are arranged on two opposite side walls of the sampling end.

9. The sample collection and detection card according to claim 1, wherein the fixing structure comprises:
a second limit insert block capable of being inserted into an inner side of the upper cover and a second elastic inverted buckle in a hook-and-buckle fit with the inner side of the upper cover which are arranged on two opposite side walls of the sampling end.

10. The sample collection and detection card according to claim 1, wherein a top of the sample loading chamber is flush with or lower than a top surface of the upper cover.
